# EUROPEAN PATENT SPECIFICATION

(11) **EP 4 748 303 B1**
(45) Date of publication and mention of the grant of the patent: **19.08.2026**
(21) Application number: 25159864.5
(22) Date of filing: 25.02.2025
(51) Int. Cl.: A61B 5/024, A61B 5/11, A61B 8/08, A61B 8/02

(54) **GUARDIAN DEVICE**
WÄCHTERVORRICHTUNG
DISPOSITIF DE GARDIEN

(30) Priority: 22.11.2024 CN 202411692845
(43) Date of publication of application: 27.05.2026
(73) Proprietor: Yuan, Yuan, Shenzhen, Guangdong (CN)
(72) Inventor: Yuan, Yuan, Shenzhen, Guangdong (CN)
(74) Representative: Herrero & Asociados, S.L.

(56) References cited:
- CN-U- 206 214 089
- CN-U- 208 677 376
- US-A1- 2018 000 405

## Description

### TECHNICAL FIELD

This application relates to the field of monitoring technologies, in particular to a guardian device.

### BACKGROUND

Since a guardian device may share the caring pressure for a guardian, the guardian device is increasingly popular among families or infant caring institutions. The guardian device includes a parent device and an infant device, which can communicate via short range wireless distance. The infant device is placed near the infant to acquire the infant's state information. The infant device transmits the infant's state information to the parent device, and the guardian may acquire the infant's state from the parent device. In the related art, the guardian device is only configured to monitor the infant's state, is restricting the application scenarios and causing the limitations on the diverse usage of users.

CN208677376U discloses multi-functional physiology information monitoring area and physiology information monitoring system. A multi -functional physiology information monitoring area, includes: flexible band, at least one sensor unit, signal pre -processing module, mode switch module, signal processing module and the control unit, signal processing module includes foetus guardianship module and infant sleep guardianship module, and foetus guardianship module is used for isolating the physiological signal of sign foetus sign based on data signal, and infant sleep guardianship module is used for isolating the physiological signal of sign baby sign based on data signal, the control unit and signal processing module are connected for obtain physiology information based on the physiological signal of sign foetus sign or the physiological signal of sign baby sign.

CN206214089U discloses a multi -functional baby's monitor, includes shell, switch, its characterized in that: be equipped with the CPU master control in the shell, the CPU master control links respectively through the serial ports has sound receiver, fetal heart monitoring device, prenatal guidance audio player, camera, be equipped with RAM card draw -in groove, charging socket, switch, earplug jack on the shell, the monitor still is furnished with the charger,

US2018/000405A1 discloses a system for monitoring health parameters of a user includes a housing including: a plurality of sensors disposed on an outer surface of the housing or within the housing for measuring a plurality of parameters of interest; a processor disposed in the housing and communicatively coupled to the plurality of sensors; a coupling element on the housing for coupling the housing to an accessory; and an accessory identifier positioned on or within the housing and communicatively coupled to the processor.

### SUMMARY

The present disclosure provides a guardian device to solve a problem that the application scenarios of the guardian device in the related art is limited. The present invention is defined in the appended set of claims.

In the guardian device provided in the present disclosure, the monitoring apparatus includes the first acquisition unit and the second acquisition unit. The first acquisition unit is configured to acquire state information of the fetus, so that the guardian device may be configured to monitor the state of the fetus. The second acquisition unit is configured to acquire the state information of the infant, and the receiving apparatus may be configured to receive the state information of the infant, so that the guardian device may be configured to monitor the state of the infant. Furthermore, the first acquisition unit or the second acquisition unit may be either selectively activated, thus the first acquisition unit and the second acquisition unit may work independently. The guardian device is configured to monitor the state of both infant and fetus. The guardian device may be used from the pregnancy period to the growth period of infant after birth, this expands the application scenarios of guardian device and meets the user diverse needs.

### BRIEF DESCRIPTION OF THE DRAWINGS

To more clearly illustrate the technical solutions in the embodiments of the present disclosure, the drawings used for description of some embodiments are described. Apparently, the drawings in the following description only illustrate some embodiments of the present disclosure. For those skilled in the art, other drawings may be acquired according to the drawings without any creative work.
FIG. 1 is a framework schematic diagram of an embodiment of a guardian device in the present disclosure.
FIG. 2 is a framework schematic diagram of an embodiment of a monitoring apparatus in the present disclosure.
FIG. 3 is a framework schematic diagram of another embodiment of a monitoring apparatus in the present disclosure.
FIG. 4 is a framework schematic diagram of an embodiment of a first acquisition unit in the present disclosure.
FIG. 5 is a framework schematic diagram of still another embodiment of a monitoring apparatus in the present disclosure.
FIG. 6 is a framework schematic diagram of further another embodiment of a monitoring apparatus in the present disclosure.
FIG. 7 is a framework schematic diagram of an embodiment of a first output unit in the present disclosure.
FIG. 8 is a framework schematic diagram of an embodiment of a receiving apparatus in the present disclosure.
FIG. 9 is a framework schematic diagram of an embodiment of a second output unit in the present disclosure.
FIG. 10 is a framework schematic diagram of an embodiment of a second acquisition unit in the present disclosure.
FIG. 11 is a framework schematic diagram of another embodiment of a receiving apparatus in the present disclosure.

### DETAILED DESCRIPTIONS

The technical solutions of the present disclosure are described in conjunction with the drawings and embodiments. It should be noted that the following embodiments are only used to illustrate the present disclosure, but do not limit the scope of the present disclosure. Similarly, the following embodiments are only some embodiments of the present disclosure and not all embodiments. All other embodiments acquired by those skilled in the art based on the embodiments in the present disclosure without the creative work are all within the scope of the present disclosure.

In embodiments of the present disclosure, a term "multiple" means at least two, such as two, or three, etc., unless otherwise specifically defined. Terms "first", "second" and "third" in embodiments of the present disclosure are only used for description purposes and cannot be understood as indicating or implying relative importance or implicitly indicating the number of technical features indicated. Thus, features defined as "first", "second", and "third" may explicitly or implicitly include at least one of the features. All directional indications (such as above, lower, left, right, front, rear...) used in embodiments of the present disclosure are only used to explain relative position relationship, motion situation, etc. between components in a specific posture (as shown in the drawings). When the specific posture changes, the directional indication also changes accordingly. Terms "include" and "have", and any modification thereof are intended to cover un-exclusive inclusion. For example, a process, a method, a system, a product, or a device that includes a series of operations or units is not limited to the listed operations or units, but optionally also includes operations or units not listed, or optionally includes other operations or units inherent to the process, method, product, or device.

"Embodiments" mentioned in the present disclosure means that specific features, structures, or characteristics described in conjunction with embodiments may be included in at least one embodiment of the present disclosure. Some embodiments including a phrase appearing in various positions in the specification does not necessarily refer to the same embodiment, and are not independents or alternative embodiments that are mutually exclusive with other embodiments. Those skilled in the art explicitly and implicitly understand that the embodiments described in the present disclosure may be combined with other embodiments.

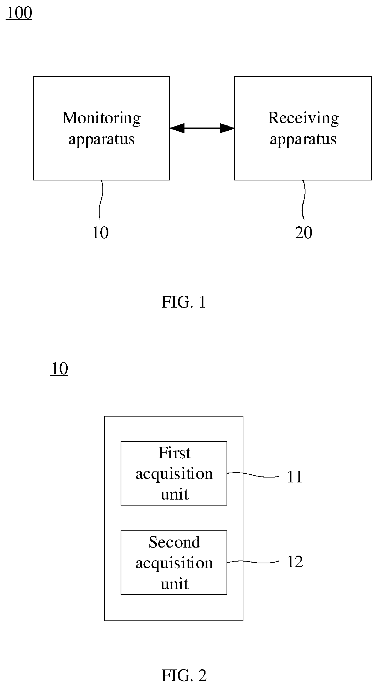

Some embodiments of the present disclosure provide a guardian device. As shown in FIG. 1, a guardian device 100 may include a monitoring apparatus 10 and a receiving apparatus 20. The monitoring apparatus 10 is configured to acquire monitoring information. The receiving apparatus 20 and the monitoring apparatus 10 are in communication connection, so that data may be transmitted between the monitoring apparatus 10 and the receiving apparatus 20. The receiving apparatus 20 may be a parent device. The receiving apparatus 20 may be a terminal device, such as a mobile phone, a handheld computer, a laptop, or a server, etc. The receiving apparatus 20 may include both a parent device and multiple terminal devices.

As shown in FIG. 2, the monitoring apparatus 10 includes a first acquisition unit 11 and a second acquisition unit 12. The first acquisition unit 11 is configured to acquire state information of the fetus when the first acquisition unit 11 is near the skin of the pregnant woman, making it convenient for the user to understand the health state of the fetus. The user may upload the joyful information of newborn state to social media and share with family and friends. The second acquisition unit 12 is configured to acquire the state information of the infant when the second acquisition unit 12 is near the infant. The receiving apparatus 20 may receive the state information of the infant, so that the guardian may acquire the state information of the infant from the receiving apparatus 20. Since the receiving apparatus 20 and the monitoring apparatus 10 are in communication connection, there may be a certain distance between the receiving apparatus 20 and the monitoring apparatus 10. The guardian carrying the receiving apparatus 20 may temporarily leave the infant, thereby reducing the guardian's caring pressure.

The monitoring apparatus 10 may be configured to selectively activate either the first acquisition unit 11 or the second acquisition unit 12. That is, the monitoring apparatus 10 may control one of the first acquisition unit 11 and the second acquisition unit 12 to be in the working state and the other to be in a non-working state, so that the first acquisition unit 11 and the second acquisition unit 12 may work independently.

In the guardian device 100 of some embodiments of the present disclosure, the monitoring apparatus 10 includes the first acquisition unit 11 and the second acquisition unit 12. The first acquisition unit 11 is configured to acquire state information of the fetus, so that the guardian device 100 may be configured to monitor the state of the fetus. The second acquisition unit 12 is configured to acquire the state information of the infant, and the receiving apparatus 20 may be configured to receive the state information of the infant, so that the guardian device 100 may be configured to monitor the state of the infant. Furthermore, the first acquisition unit 11 or the second acquisition unit 12 may be either selectively activated, so that the first acquisition unit 11 and the second acquisition unit 12 may work independently. The guardian device 100 is configured to monitor the state of both infant and fetus. The guardian device 100 may be used from the pregnancy period to the growth period of infant after birth, this expands the application scenarios of guardian device and meets the user diverse needs.

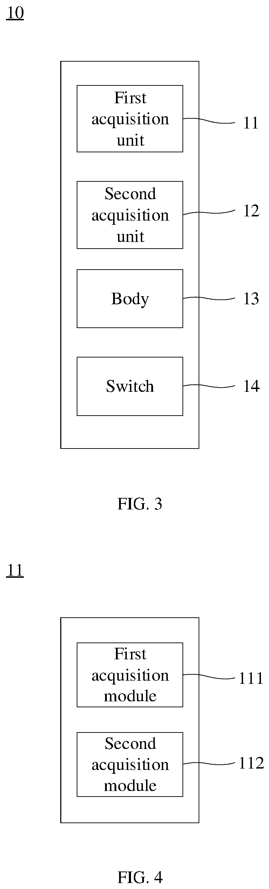

In some embodiments, as shown in FIG. 3, the monitoring apparatus 10 includes a body 13. The body 13 may include a housing and a control motherboard located within the housing, and the housing serves as a carrier for other components. The first acquisition unit 11 and the second acquisition unit 12 are both fixedly arranged on the body 13. According to different types of sensor, the acquisition unit may be arranged inside the body 13, or a portion of the acquisition unit may be arranged inside the body 13 while another portion of the acquisition unit is located on the surface of the body 13 to facilitate the acquisition of corresponding state information. The first acquisition unit 11 and the second acquisition unit 12 are fixedly arranged on the body 13, so that both the first acquisition unit 11 and the second acquisition unit 12 are integrated into the body 13. The first acquisition unit 11, the second acquisition unit 12, and the body 13 form an integrated structure. On the one hand, the first acquisition unit 11 and the second acquisition unit 12 may share some components of the body 13, such as the housing and control motherboard, thereby reducing the volume and cost of the monitoring apparatus 10. On the other hand, the monitoring apparatus 10 does not require assembly and connection operations during use, thereby enhancing the convenience of use. On the still other hand, the monitoring apparatus 10 has good integrity and may be conveniently stored. The monitoring apparatus 10 may include a switch 14 configured to selectively activate either the first acquisition unit 11 or the second acquisition unit 12, enabling the guardian device 100 to switch guardian modes in different usage scenarios, so that the first acquisition unit 11 and the second acquisition unit 12 may work independently. The switch 14 may be arranged on the body 13 or on the acquisition unit. The switch 14 may be a touch switch, a push-pull switch, or a push switch.

In some embodiments, at least one of the first acquisition unit 11 and the second acquisition unit 12 is detachably connected to the body 13. That is, one of the first acquisition unit 11 and the second acquisition unit 12 is detachably connected to the body 13, or both the first acquisition unit 11 and the second acquisition unit 12 are detachably connected to the body 13. The acquisition unit may be independent of the body 13. The acquisition unit may be assembled and connected to the body 13 through methods such as clamping connection, bonding connection, threaded connection, or magnetic connection. The acquisition unit may be detached from the body 13. In some embodiments, the acquisition unit may be connected to the body 13 through a data wire, and data may be transmitted between the acquisition unit and the body 13, so that no assembled connection is required between the acquisition unit and the body 13. When the data wire is removed, the acquisition unit may be detached from the body 13. In this way, the acquisition unit may be arranged separately from the body 13. When the usage scenario of the guardian device 100 changes, it is convenient to remove the acquisition unit that is temporarily not needed from the body 13, which is beneficial for the first acquisition unit 11 and the second acquisition unit 12 to work independently. For example, during the pregnancy period of the fetus , since the second acquisition unit 12 is not needed, the second acquisition unit 12 may be detached from the body 13. For example, during the growth period of the fetus after birth, since the first acquisition unit 11 is not needed, the first acquisition unit 11 may be detached from the body 13. In addition, the first acquisition unit 11 and the second acquisition unit 12 may share some components of the body 13, such as the control motherboard, thereby reducing the cost of the monitoring apparatus 10.

As shown in FIG. 4, in some embodiments, the first acquisition unit 11 includes a first acquisition module 111, and the first acquisition module 111 is configured to acquire heart rate information of the fetus when the first acquisition unit 11 is near the skin of the pregnant woman. The heart rate of the fetus is the heartbeat of the fetus, which may be stably detected after sixteen weeks of pregnancy. The heart rate of the fetus is an important indicator for judging vital signs of the fetus. The first acquisition module 111 may acquire heart rate information of the fetus, so that the user understands the growth and development of the fetus at any time.

In some embodiments, the first acquisition module 111 includes one of an ultrasonic transducer and a pressure sensor. The ultrasonic transducer may generate ultrasound beams, a portion of the incident sound beam reaches the surface of the heart of the fetus. Due to the Doppler effect, the ultrasound frequency shifts, and the reflected signal may be separated and amplified to acquire heart rate information of the fetus. The pressure sensor may be a piezoelectric thin film sensor, which may sense the mechanical vibration of the pregnant woman's abdominal skin caused by the heartbeat of the fetus, and convert the mechanical vibration into an electrical signal. The electrical signal may be amplified, filtered, analog-to-digital converted, separated, and processed in other methods to acquire heart rate information of the fetus.

As shown in FIG. 4, in some embodiments, the first acquisition unit 11 includes a second acquisition module 112, the second acquisition module 112 is configured to acquire movement information of the fetus when the first acquisition unit 11 is near the skin of the pregnant woman. The movement of the fetus is the first objective sign of life of the fetus, and the pregnant women may feel the movement of the fetus at around four months of pregnancy. The number, speed, and intensity of the movement of the fetus indicate the health state of the fetus in the mother's body. The second acquisition module 112 may acquire movement information of the fetus, so that the user to understand the growth and development of the fetus at any time.

The first acquisition unit 11 may includes one of the first acquisition module 111 and the second acquisition module 112. In some embodiments, the first acquisition unit 11 may include both the first acquisition module 111 and the second acquisition module 112.

In some embodiments, the second acquisition module 112 includes at least one of an ultrasound transducer, a pressure sensor, and an acceleration sensor. The ultrasonic transducer may acquire movement information of the fetus based on the Doppler effect, the pressure sensor and the acceleration sensor may acquire movement information of the fetus based on mechanical vibration. When the second acquisition module 112 includes two or more of the ultrasonic transducer, the pressure sensor, and the acceleration sensor, multi-scale detection through multiple sensors may improve the accuracy of detection of the movement information of the fetus.

When the arrangement of sensors of the first acquisition module 111 is the same as that of the second acquisition module 112, the first acquisition module 111 may share some of the sensors with the second acquisition module 112, that is, the first acquisition module 111 (or the second acquisition module 112) may be configured to acquire both heart rate information and movement information of the fetus, thereby achieving reuse of the sensors and reducing the number of components in the monitoring apparatus 10.

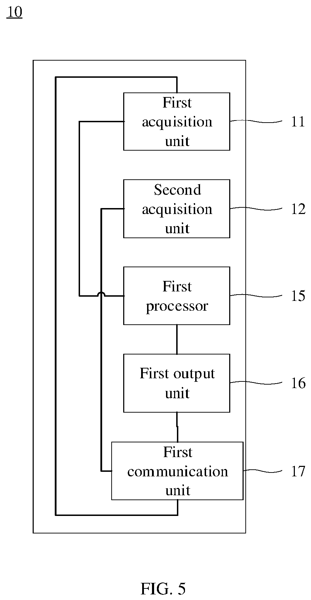

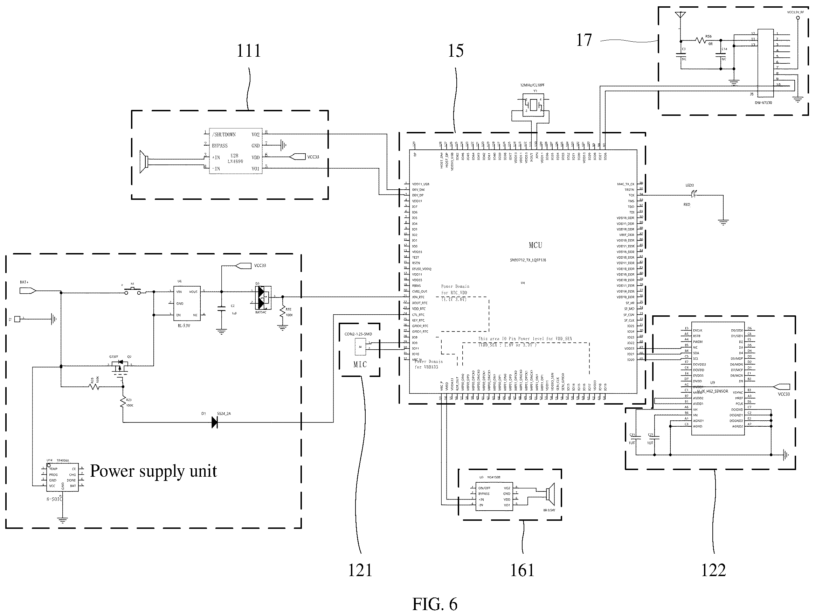

The state information of the fetus acquired by the first acquisition unit 11 may be outputted at the end of the monitoring apparatus 10. As shown in FIGs. 5 and 6, in some embodiments, the monitoring apparatus 10 includes a first processor 15 and a first output unit 16. The first processor 15 may be a general-purpose processor, a Digital Signal Processor (DSP), an Application Specific Integrated Circuit (ASIC), a Field Programmable Gate Array (FPGA), or other programmable logic devices, discrete gates or transistor logic devices, or discrete hardware components. The general-purpose processor may be a microprocessor to reduce the size of the first processor 15, which is beneficial for the miniaturization of the monitoring apparatus 10. The first processor 15 is electrically connected to the first acquisition unit 11, and is configured to generate the first output data based on the state information of the fetus. The state information of the fetus acquired by the first acquisition unit 11 is a vibration signal. Since it is difficult for the user to understand the information represented by the vibration signal, the state information is processed to generate the first output data, so that the outputted state information is converted into a form which is easy for the user to understand , thereby improving the usability of the product. The first output unit 16 is electrically connected to the first processor 15, and is configured to receive and play the first output data, so that the monitoring apparatus 10 may not only monitor the state of the fetus, but also enable the user to share the joy of new life with family and friends through the monitoring apparatus 10. In addition, since the state information of the fetus is directly outputted at the end of the monitoring apparatus 10, the output of the state information of the fetus does not depend on the receiving apparatus 20, thereby reducing the amount of data transmission between the monitoring apparatus 10 and the receiving apparatus 20.

In some embodiments, the first output data includes prompt voice configured to indicate the health state of the fetus. For example, the first processor 15 processes the heart rate information of the fetus to generate the prompt voice, and the prompt voice may indicate to the user whether the heart rate of the fetus is within the normal range. The heart rate of the fetus changes constantly with the different uterus environment. The change of heart rate of the fetus indicates a normal regulatory function of the central nervous system and a good condition of the fetus in the uterus. The normal heart rate of the fetus is 110-160 beats per minute. If the heart rate of the fetus is less than 100 beats per minute or greater than 160 beats per minute, and lasts for more than 10 minutes, the heart rate of the fetus is called bradycardia or tachycardia. If the first processor 15 detects bradycardia or tachycardia when processing heart rate information of the fetus, the first processor 15 may generate the prompt voice, so that the user detects abnormal situations in time.

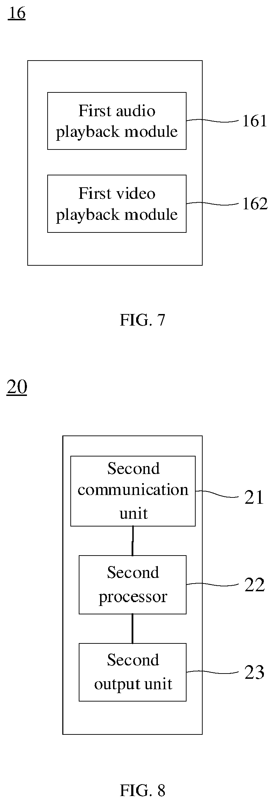

In some embodiments, the state information of the fetus includes a vibration signal of heart rate of the fetus or vibration signal of movement of the fetus, and the first output data includes audio data generated based on the vibration signal of the heart rate of the fetus or the vibration signal of the movement of the fetus, so that the user may intuitively feel the heart rate of the fetus or the movement of the fetus through sound. As shown in FIG. 7, the first output unit 16 includes a first audio playback module 161 configured to receive and play audio data. The first audio playback module 161 may include a speaker, so that the first audio playback module 161 may output the vibration signal of the heart rate of the fetus or the vibration signal of the movement of the fetus in the form of sound. The vibration signal of the heart rate of the fetus or the vibration signal of the movement of the fetus is converted into audio data for playback, so that the presentation of state information of the fetus is more intuitive, thereby enhancing the user experience.

In some embodiments, the state information of the fetus includes the vibration signal of the heart rate of the fetus or the vibration signal of the movement of the fetus, and the first output data includes video data generated based on the vibration signal of the heart rate of the fetus or the vibration signal of the movement of the fetus, so that the user may intuitively feel the heart rate of the fetus or the movement of the fetus through images. As shown in FIG. 7, the first output unit 16 includes a first video playback module 162 configured to receive and play video data. The first video playback module 162 may include a display, so that the first video playback module 162 may output the vibration signal of the heart rate of the fetus or the vibration signal of the movement of the fetus in the form of images. For example, the video data may be a spectral waveform diagram, so that the state information of the fetus is visualized. The vibration signal of the heart rate of the fetus or the vibration signal of the movement of the fetus is converted into video data for playback, so that the presentation of state information of the fetus is more intuitive, thereby enhancing the user experience.

The first output unit 16 may include one of the first audio playback module 161 and the first video playback module 162. In some embodiments, the first output unit 16 may includes both the first audio playback module 161 and the first video playback module 162.

The state information of the fetus acquired by the first acquisition unit 11 may be outputted at the end of the receiving apparatus 20. As shown in FIG. 5, in some embodiments, the monitoring apparatus 10 includes a first communication unit 17 electrically connected to the first acquisition unit 11 and the second acquisition unit 12. The first communication unit 17 is configured to transmit the state information of the fetus and the state information of the infant to the receiving apparatus 20, so that both the state information of the fetus and the state information of the infant may be outputted at the end of the receiving apparatus 20. Since the state information of the fetus is outputted at the end of the receiving apparatus 20, some components may be not arranged at the end of the monitoring apparatus 10. For example, the end of the monitoring apparatus 10 may not be arranged with a speaker or a display, thereby reducing the number of components and the volume of the monitoring apparatus 10.

As shown in FIG. 8, in some embodiments, the receiving apparatus 20 includes a second communication unit 21, a second processor 22, and a second output unit 23. The second communication unit 21 and the first communication unit 17 are in communication connection, and the second communication unit 21 is configured to receive the state information of the fetus and the state information of the infant. The communication connection between the second communication unit 21 and the first communication unit 17 may be a wired connection. For example, when the receiving apparatus 20 is a computer, the second communication unit 21 may be connected to the first communication unit 17 through a data wire. The communication connection between the second communication unit 21 and the first communication unit 17 may be a wireless connection. For example, when the receiving apparatus 20 is a parent device, the communication unit may include one or more short-distance communication modules including a WiFi communication module, a Bluetooth communication module, and a ZigBee communication module, etc. In this way, the second communication unit 21 may communicate with the first communication unit 17 in a short-distance, and the maximum communication distance between the monitoring apparatus 10 and the receiving apparatus 20 may be between 600-800 meters. For another example, when the receiving apparatus 20 is a mobile phone, the communication connection between the second communication unit 21 and the first communication unit 17 may be implemented through the Internet. For example, the first communication unit 17 uploads the state information of the fetus and the state information of the infant to the Internet, and the second communication unit 21 may acquire the state information from the Internet, so that the user may share the joy of new life with family and friends who are not around. The second processor 22 is electrically connected to the second communication unit 21, and is configured to generate second output data based on state information of the fetus. The second output unit 23 is electrically connected to the second processor 22, and is configured to receive and play the second output data. The arrangement of the second processor 22 may refer to the arrangement of the first processor 15, and the arrangement of the second output unit 23 may refer to the arrangement of the first output unit 16.

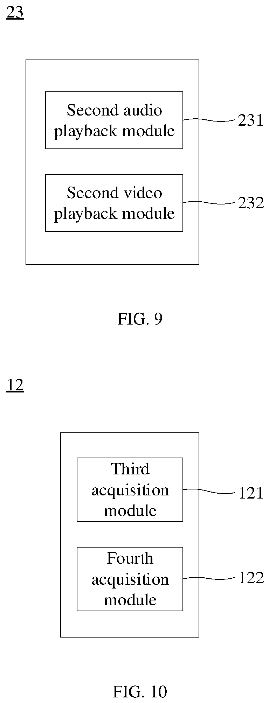

In some embodiments, the state information of the fetus includes the vibration signal of the heart rate of the fetus or the vibration signal of the movement of the fetus, and the second output data includes audio data generated based on the vibration signal of the heart rate of the fetus or the vibration signal of the movement of the fetus. The second output unit 23 includes a second audio playback module 231, as shown in FIG. 9, the second audio playback module 231 is configured to receive and play the audio data. The second audio playback module 231 may include a speaker, so that the vibration signal of the heart rate of the fetus or the vibration signal of the movement of the fetus is converted into the audio data for playback, making the presentation of state information of the fetus more intuitive.

In some embodiments, the state information of the fetus includes the vibration signal of the heart rate of the fetus or the vibration signal of the movement of the fetus, and the second output data includes video data generated based on the vibration signal of the heart rate of the fetus or the vibration signal of the movement of the fetus. The second output unit 23 includes a second video playback module 232, as shown in FIG. 9, the second video playback module 232 is configured to receive and play the video data. The second video playback module 232 may include a display, so that the vibration signal of the heart rate of the fetus or the vibration signal of the movement of the fetus is converted into the video data for playback, making the presentation of state information of the fetus more intuitive.

The second output unit 23 may includes one of the second audio playback module 231 and the second video playback module 232. In some embodiments, the second output unit 23 may includes both the second audio playback module 231 and the second video playback module 232.

In some embodiments, the state information of the infant may include sound information and video information, so that the guardian may understand the infant's state. As shown in FIG. 10, the second acquisition unit 12 includes a third acquisition module 121 and a fourth acquisition module 122. The third acquisition module 121 is configured to acquire the sound information of the infant when the second acquisition unit 12 is near the infant. For example, the third acquisition module 121 may acquire the infant's breathing sounds and crying sounds, etc. The fourth acquisition module 122 is configured to acquire the video information of the infant when the second acquisition unit 12 is near the infant. The second output unit 23 includes a second audio playback module 231 and a second video playback module 232. The second audio playback module 231 is configured to receive and play the sound information, and the second video playback module 232 is configured to receive and play the video information. By acquiring the sound information and video information of the infant, the monitoring apparatus 10 helps the guardian to have a comprehensive understanding of the condition of the infant.

In some embodiments, the third acquisition module 121 includes a microphone, and the fourth acquisition module 122 includes a camera. When the infant makes a sound, such as crying, the sound will cause a diaphragm in the microphone to vibrate, so that the microphone generates the sound information in the form of an electrical signal. The camera may acquire the video information of the infant and convert the video information into a digital signal for transmission.

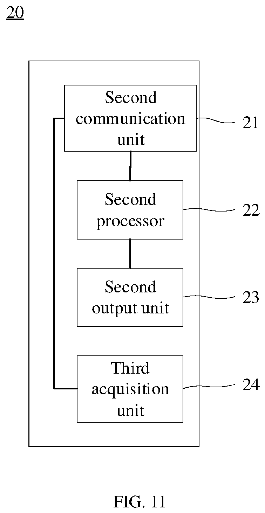

In some embodiments, as shown in FIG. 11, the receiving apparatus 20 includes a third acquisition unit 24 configured to acquire audio feedback. The third acquisition unit 24 may include a microphone, and the audio feedback may be a voice of the guardian which is configured to soothe the infant. The third acquisition unit 24 and the second communication unit 21 are in communication connection, and the second communication unit 21 is configured to transmit the audio feedback to the first communication unit 17. The monitoring apparatus 10 includes a first audio playback module 161 electrically connected to the first communication unit 17. The first audio playback module 161 is configured to play the audio feedback to soothe the infant. Since the monitoring apparatus 10 plays the audio feedback, the infant may receive the voice of the guardian, thereby enhancing the infant's security feeling and helping stabilize emotion of the infant.

The monitoring apparatus 10 and the receiving apparatus 20 may include a power supply unit to provide electrical energy for the monitoring apparatus 10 and the receiving apparatus 20 during operation. The power supply unit may be an external power source or a built-in battery.

The above are only some embodiments of the present disclosure and do not limit the scope of the present disclosure.

## Claims

1. A guardian device (100), comprising:
a monitoring apparatus (10), comprising a first acquisition unit (11) and a second acquisition unit (12); and
a receiving apparatus (20), wherein the receiving apparatus (20) and the monitoring apparatus (10) are in communication connection;
wherein the first acquisition unit (11) is configured to acquire state information of a fetus when the first acquisition unit (11) is near skin of a pregnant woman, the second acquisition unit (12) is configured to acquire state information of an infant when the second acquisition unit (12) is near the infant, and the receiving apparatus (20) is configured to receive the state information of the infant;
wherein the monitoring apparatus (10) is configured to selectively activate either the first acquisition unit (11) or the second acquisition unit (12);
wherein the monitoring apparatus (10) comprises a first communication unit (17) electrically connected to the first acquisition unit (11) and the second acquisition unit (12), and the first communication unit (17) is configured to transmit the state information of the fetus and the state information of the infant to the receiving apparatus (20);
wherein the state information of the fetus comprises a vibration signal of the heart rate of the fetus; and
wherein the receiving apparatus (20) comprises:
a second communication unit (21), wherein the second communication unit (21) and the first communication unit (17) are in communication connection, and the second communication unit (21) is configured to receive the state information of the fetus and the state information of the infant;
a second processor (22), electrically connected to the second communication unit (21), and configured to generate second output data based on the state information of the fetus, wherein the second output data comprises audio data generated by converting the vibration signal of the heart rate of the fetus into audio data for playback; and
a second output unit (23), electrically connected to the second processor (22), and configured to receive and play the second output data, wherein the second output unit (23) comprises a second audio playback module (231) comprising a speaker and being configured to receive and play the vibration signal of the heart rate of the fetus converted into the audio data for playback.

2. The guardian device (100) according to claim 1, wherein the monitoring apparatus (10) comprises a body (13) and a switch (14), the first acquisition unit (11) and the second acquisition unit (12) are fixedly arranged on the body (13), and the switch (14) is configured to selectively activate either the first acquisition unit (11) or the second acquisition unit (12).

3. The guardian device (100) according to claim 1, wherein the monitoring apparatus (10) comprises a body (13), and at least one of the first acquisition unit (11) and the second acquisition unit (12) is detachably connected to the body (13).

4. The guardian device (100) according to claim 1, wherein the first acquisition unit (11) comprises a first acquisition module (111), and the first acquisition module (111) is configured to acquire heart rate information of the fetus when the first acquisition unit (11) is near the skin of the pregnant woman.

5. The guardian device (100) according to claim 4, wherein the first acquisition module (111) comprises one of an ultrasonic transducer and a pressure sensor.

6. The guardian device (100) according to claim 1, wherein the first acquisition unit (11) comprises a second acquisition module (112), and the second acquisition module (112) is configured to acquire movement information of the fetus when the first acquisition unit (11) is near the skin of the pregnant woman.

7. The guardian device (100) according to claim 6, wherein the second acquisition module (112) comprises at least one of an ultrasonic transducer, a pressure sensor, and an acceleration sensor.

8. The guardian device (100) according to claim 1, wherein the monitoring apparatus (10) comprises:
a first processor (15), electrically connected to the first acquisition unit (11), and configured to generate first output data based on the state information of the fetus; and
a first output unit (16), electrically connected to the first processor (15), and configured to receive and play the first output data.

9. The guardian device (100) according to claim 8, wherein the state information of the fetus comprises a vibration signal of the heart rate of the fetus or a vibration signal of the movement of the fetus, and the first output data comprises audio data generated based on the vibration signal of the heart rate of the fetus or the vibration signal of the movement of the fetus;
the first output unit (16) comprises a first audio playback module (161) configured to receive and play the audio data;
the first audio playback module (161) comprises another speaker configured to output the vibration signal of the heart rate of the fetus in a form of sound.

10. The guardian device (100) according to claim 8, wherein the state information of the fetus comprises a vibration signal of the heart rate of the fetus or a vibration signal of the movement of the fetus, and the first output data comprises video data generated based on the vibration signal of the heart rate of the fetus or the vibration signal of the movement of the fetus;
the first output unit (16) comprises a first video playback module (162) configured to receive and play the video data.

11. The guardian device (100) according to claim 1, wherein the state information of the infant comprises sound information and video information;
the second acquisition unit (12) comprises:
a third acquisition module (121), configured to acquire the sound information of the infant when the second acquisition unit (12) is near the infant;
a fourth acquisition module (122), configured to acquire the video information of the infant when the second acquisition unit (12) is near the infant;
the second output unit (23) comprises a second audio playback module (231) and a second video playback module (232), the second audio playback module (231) is configured to receive and play the sound information, and the second video playback module (232) is configured to receive and play the video information.

12. The guardian device (100) according to claim 11, wherein the third acquisition module (121) comprises a microphone, and the fourth acquisition module (122) comprises a camera.

13. The guardian device (100) according to claim 1, wherein the receiving apparatus (20) comprises a third acquisition unit (24) configured to acquire audio feedback, the third acquisition unit (24) and the second communication unit (21) are in communication connection, and the second communication unit (21) is configured to transmit the audio feedback to the first communication unit (17);
the monitoring apparatus (10) comprises a first audio playback module (161) electrically connected to the first communication unit (17), and the first audio playback module (161) is configured to play the audio feedback to soothe the infant.

## Patentansprüche

1. Ein Wächtergerät (100), bestehend aus:
eine Überwachungsvorrichtung (10), umfassend eine erste Erfassungseinheit (11) und eine zweite Erfassungseinheit (12); und eine Empfangsvorrichtung (20), wobei die Empfangsvorrichtung (20) und die Überwachungsvorrichtung (10) in Kommunikationsverbindung stehen; wobei die erste Erfassungseinheit (11) konfiguriert ist, um Zustandsinformationen eines Fötus zu erfassen, wenn sich die erste Erfassungseinheit (11) in der Nähe der Haut einer schwangeren Frau befindet, und die zweite Erfassungseinheit (12) konfiguriert ist, um Zustandsinformationen eines Säuglings zu erfassen, wenn die zweite Erfassung erfolgt Die Überwachungsvorrichtung (12) befindet sich in der Nähe des Säuglings, und die Empfangsvorrichtung (20) ist zum Empfangen der Zustandsinformationen des Säuglings konfiguriert. Die Überwachungsvorrichtung (10) ist dazu konfiguriert, selektiv entweder die erste Erfassungseinheit (11) oder die zweite Erfassungseinheit (12) zu aktivieren Übertragen der Zustandsinformationen des Fötus und der Zustandsinformationen des Säuglings an die Empfangsvorrichtung (20) ;wobei die Zustandsinformationen des Fötus ein Vibrationssignal der Herzfrequenz des Fötus umfassen;und wobei die Empfangsvorrichtung (20) umfasst:
eine zweite Kommunikationseinheit (21), wobei die zweite Kommunikationseinheit (21) und die erste Kommunikationseinheit (17) in Kommunikationsverbindung stehen und die zweite Kommunikationseinheit (21) konfiguriert ist, um die Zustandsinformationen des Fötus und die Zustandsinformationen des Säuglings zu empfangen; einen zweiten Prozessor (22), der elektrisch mit der zweiten Kommunikationseinheit (21) verbunden ist und konfiguriert ist, um zweite Ausgabedaten basierend auf den Zustandsinformationen des 2. Fötus zu erzeugen, wobei die zweiten Ausgabedaten Audiodaten umfassen, die durch Umwandeln des Vibrationssignals des Herzens erzeugt werden Rate des Fötus in Audiodaten zur Wiedergabe; Fotus und eine zweite Ausgabeeinheit (23), die elektrisch mit dem zweiten Prozessor (22) verbunden und zum Empfangen und Abspielen der zweiten Ausgabedaten konfiguriert ist, wobei die zweite Ausgabeeinheit (23) ein zweites Audiowiedergabemodul (231) umfasst, das einen Lautsprecher umfasst und zum Empfangen und Abspielen eines Vibrationssignals der Herzfrequenz des Fötus, das zur Wiedergabe in Audiodaten umgewandelt wurde, konfiguriert ist.

2. Wächtergerät (100) nach Anspruch 1, wobei die Überwachungsvorrichtung (10) einen Körper (13) und einen Schalter (14) umfasst, die erste Erfassungseinheit (11) und die zweite Erfassungseinheit (12) fest am Körper (13) angeordnet sind und der Schalter (14) so konfiguriert ist, dass er wahlweise entweder die erste Erfassungseinheit (11) oder die zweite Erfassungseinheit (12) aktiviert.

3. Das Überwachungsgerät (100) nach Anspruch 1, wobei das Überwachungsgerät (10) einen Körper (13) und mindestens eine der ersten Erfassungseinheit (11) und der zweiten Erfassungseinheit (12) umfasst.

4. Das Schutzgerät (100) nach Anspruch 1, wobei die erste Erfassungseinheit (11) ein erstes Erfassungsmodul (111) umfasst und das erste Erfassungsmodul (111) so konfiguriert ist, dass es Herzfrequenzinformationen des Fötus erfasst, wenn sich die erste Erfassungseinheit (11) in der Nähe der Haut der schwangeren Frau befindet.

5. Wächtergerät (100) nach Anspruch 4, wobei das erste Erfassungsmodul (111) einen Ultraschallwandler oder einen Drucksensor umfasst.

6. Das Schutzgerät (100) nach Anspruch 1, wobei die erste Erfassungseinheit (11) ein zweites Erfassungsmodul (112) umfasst und das zweite Erfassungsmodul (112) so konfiguriert ist, dass es Bewegungsinformationen des Fötus erfasst, wenn sich die erste Erfassungseinheit (11) in der Nähe der Haut der schwangeren Frau befindet.

7. Das Wächtergerät (100) nach Anspruch 6, wobei das zweite Erfassungsmodul (112) umfasst mindestens einen Ultraschallwandler, einen Drucksensor und einen Beschleunigungssensor.

8. Das Wächtergerät (100) nach Anspruch 1, wobei das Überwachungsgerät (10) Folgendes umfasst: einen ersten Prozessor (15), der elektrisch mit der ersten Erfassungseinheit (11) verbunden ist und dazu konfiguriert ist, erste Ausgabedaten basierend auf den Zustandsinformationen des Fötus zu erzeugen; und eine erste Ausgabeeinheit (16), die elektrisch mit dem ersten Prozessor (15) verbunden ist und dazu konfiguriert ist, die ersten Ausgabedaten zu empfangen und wiederzugeben.

9. Wächtergerät (100) nach Anspruch 8, wobei die Zustandsinformationen des Fötus ein Vibrationssignal der Herzfrequenz des Fötus oder ein Vibrationssignal der Bewegung des Fötus umfassen und die ersten Ausgabedaten Audiodaten umfassen, die auf der Grundlage des Vibrationssignals der Herzfrequenz des Fötus oder des Vibrationssignals der Bewegung des Fötus erzeugt wurden; die erste Ausgabeeinheit (16) umfasst ein erstes Audiowiedergabemodul (161), das zum Empfangen und Abspielen konfiguriert ist Audiodaten; das erste Audiowiedergabemodul (161) umfasst einen weiteren Lautsprecher, der dazu konfiguriert ist, das Vibrationssignal der Herzfrequenz des Fötus in Form von Ton auszugeben.

10. Wächtergerät (100) nach Anspruch 8, wobei die Zustandsinformationen des Fötus ein Vibrationssignal der Herzfrequenz des Fötus oder ein Vibrationssignal der Bewegung des Fötus umfassen und die ersten Ausgabedaten Videodaten umfassen, die auf der Grundlage des Vibrationssignals der Herzfrequenz des Fötus oder des Vibrationssignals der Bewegung des Fötus erzeugt wurden; die erste Ausgabeeinheit (16) umfasst ein erstes Videowiedergabemodul (162), das zum Empfangen und Abspielen konfiguriert ist die Videodaten.

11. Das Wächtergerät (100) nach Anspruch 1, wobei die Zustandsinformationen des Säuglings Toninformationen und Videoinformationen umfassen; die zweite Erfassungseinheit (12) umfasst: ein drittes Erfassungsmodul (121), das so konfiguriert ist, dass es die Toninformationen des Säuglings erfasst, wenn sich die zweite Erfassungseinheit (12) in der Nähe des Säuglings befindet; ein viertes Erfassungsmodul (122), das so konfiguriert ist, dass es die Videoinformationen des Säuglings erfasst, wenn sich die zweite Erfassungseinheit (12) in der Nähe des Säuglings befindet; die zweite Ausgabeeinheit (23) umfasst ein zweites Audiowiedergabemodul (231) und ein zweites Videowiedergabemodul (232), das zweite Audiowiedergabemodul (231) ist so konfiguriert, dass es die Toninformationen empfängt und wiedergibt, und das zweite Videowiedergabemodul (232) ist so konfiguriert, dass es die Videoinformationen empfängt und wiedergibt.

12. Das Wächtergerät (100) nach Anspruch 11, wobei das dritte Erfassungsmodul (121) umfasst ein Mikrofon und das vierte Erfassungsmodul (122) umfasst eine Kamera.

13. Wächtergerät (100) nach Anspruch 1, wobei die Empfangsvorrichtung (20) eine dritte Erfassungseinheit (24) umfasst, die zum Erfassen von Audio-Feedback konfiguriert ist, die dritte Erfassungseinheit (24) und die zweite Kommunikationseinheit (21) in Kommunikationsverbindung stehen und die zweite Kommunikationseinheit (21) zum Übertragen des Audio-Feedbacks an die erste Kommunikationseinheit (17) konfiguriert ist; die Überwachungsvorrichtung (10) eine erste Audio-Wiedergabe umfasst Das Modul (161) ist elektrisch mit der ersten Kommunikationseinheit (17) verbunden und das erste Audiowiedergabemodul (161) ist dazu konfiguriert, das Audio-Feedback abzuspielen, um den Säugling zu beruhigen.

## Revendications

1. Un dispositif de surveillance (100), **caractérisé en ce qu'**il comprend :un appareil de surveillance (10) comprenant une première unité d'acquisition (11) et une deuxième unité d'acquisition (12) ;un appareil de réception (20) en communication avec l'appareil de surveillance (10) ;la première unité d'acquisition (11) étant configurée pour acquérir des informations d'état d'un fœtus lorsqu'elle se trouve à proximité de la peau d'une femme enceinte ;
la deuxième unité d'acquisition (12) étant configurée pour acquérir des informations d'état d'un nourrisson lorsqu'elle se trouve à proximité du nourrisson ;
l'appareil de réception (20) étant configuré pour recevoir lesdites informations d'état ;
l'appareil de surveillance (10) étant configuré pour activer sélectivement soit la première unité d'acquisition (11), soit la deuxième unité d'acquisition (12), et pour transmettre les informations d'état du fœtus et les informations d'état du nourrisson à l'appareil de réception (20) ;les informations d'état du fœtus comprenant un signal vibratoire de la fréquence cardiaque fœtale ;et l'appareil de réception (20) comprenant :une deuxième unité de communication (21) en communication avec une première unité de communication (17), configurée pour recevoir les informations d'état du fœtus et les informations d'état du nourrisson ;un deuxième processeur (22) connecté électriquement à la deuxième unité de communication (21), configuré pour générer des deuxièmes données de sortie à partir des informations d'état du fœtus, les deuxièmes données de sortie comprenant des données audio obtenues par conversion du signal vibratoire de la fréquence cardiaque fœtale en données audio lisibles ;une deuxième unité de sortie (23) connectée électriquement au deuxième processeur (22), configurée pour recevoir et restituer les deuxièmes données de sortie ; la deuxième unité de sortie (23) comprenant un deuxième module de lecture audio (231) muni d'un haut-parleur, configuré pour recevoir et restituer le signal vibratoire de la fréquence cardiaque fœtale converti en données audio.

2. Dispositif de surveillance (100) selon la revendication 1, **caractérisé en ce que** l'appareil de surveillance (10) comprend un corps (13) et un commutateur (14), la première unité d'acquisition (11) et la deuxième unité d'acquisition (12) étant fixées sur le corps (13), et le commutateur (14) étant configuré pour activer sélectivement soit la première unité d'acquisition (11), soit la deuxième unité d'acquisition (12).

3. Dispositif de surveillance (100) selon la revendication 1, **caractérisé en ce que** l'appareil de surveillance (10) comprend un corps (13) et au moins l'une parmi la première unité d'acquisition (11) et la deuxième unité d'acquisition (12).

4. Dispositif de surveillance (100) selon la revendication 1, **caractérisé en ce que** la première unité d'acquisition (11) comprend un premier module d'acquisition (111) configuré pour acquérir des informations de fréquence cardiaque fœtale lorsque la première unité d'acquisition (11) se trouve à proximité de la peau de la femme enceinte.

5. Dispositif de surveillance (100) selon la revendication 4, **caractérisé en ce que** le premier module d'acquisition (111) comprend un transducteur ultrasonore ou un capteur de pression.

6. Dispositif de surveillance (100) selon la revendication 1, **caractérisé en ce que** la première unité d'acquisition (11) comprend un deuxième module d'acquisition (112) configuré pour acquérir des informations de mouvement fœtal lorsque la première unité d'acquisition (11) se trouve à proximité de la peau de la femme enceinte.

7. Dispositif de surveillance (100) selon la revendication 6, **caractérisé en ce que** le deuxième module d'acquisition (112) comprend au moins l'un parmi : un transducteur ultrasonore, un capteur de pression et un accéléromètre.

8. Dispositif de surveillance (100) selon la revendication 1, **caractérisé en ce que** l'appareil de surveillance (10) comprend :
un premier processeur (15) connecté électriquement à la première unité d'acquisition (11), configuré pour générer des premières données de sortie à partir des informations d'état du fœtus ;une première unité de sortie (16) connectée électriquement au premier processeur (15), configurée pour recevoir et restituer les premières données de sortie.

9. Dispositif de surveillance (100) selon la revendication 8, **caractérisé en ce que** les informations d'état du fœtus comprennent un signal vibratoire de la fréquence cardiaque fœtale ou un signal vibratoire du mouvement fœtal, et les premières données de sortie comprennent des données audio générées à partir dudit signal vibratoire ;la première unité de sortie (16) comprenant un premier module de lecture audio (161) configuré pour recevoir et restituer les données audio ;le premier module de lecture audio (161) comprenant un haut-parleur supplémentaire configuré pour restituer le signal vibratoire de la fréquence cardiaque fœtale sous forme sonore.

10. Dispositif de surveillance (100) selon la revendication 8, **caractérisé en ce que** les informations d'état du fœtus comprennent un signal vibratoire de la fréquence cardiaque fœtale ou un signal vibratoire du mouvement fœtal, et les premières données de sortie comprenant des données vidéo générées à partir dudit signal vibratoire ;la première unité de sortie (16) comprenant un premier module de lecture vidéo (162) configuré pour recevoir et restituer les données vidéo.

11. Dispositif de surveillance (100) selon la revendication 1, **caractérisé en ce que** les informations d'état du nourrisson comprennent des informations sonores et des informations vidéo ;la deuxième unité d'acquisition (12) comprenant : un troisième module d'acquisition (121) configuré pour acquérir les informations sonores du nourrisson lorsque la deuxième unité d'acquisition (12) se trouve à proximité du nourrisson ;un quatrième module d'acquisition (122) configuré pour acquérir les informations vidéo du nourrisson lorsque la deuxième unité d'acquisition (12) se trouve à proximité du nourrisson ;la deuxième unité de sortie (23) comprenant un deuxième module de lecture audio (231) et un deuxième module de lecture vidéo (232), le deuxième module de lecture audio (231) étant configuré pour recevoir et restituer les informations sonores, et le deuxième module de lecture vidéo (232) étant configuré pour recevoir et restituer les informations vidéo.

12. Dispositif de surveillance (100) selon la revendication 11, **caractérisé en ce que** le troisième module d'acquisition (121) comprend un microphone et le quatrième module d'acquisition (122) comprend une caméra.

13. Dispositif de surveillance (100) selon la revendication 1, **caractérisé en ce que** l'appareil de réception (20) comprend une troisième unité d'acquisition (24) configurée pour acquérir un retour audio, la troisième unité d'acquisition (24) étant en communication avec la deuxième unité de communication (21), et la deuxième unité de communication (21) étant configurée pour transmettre ledit retour audio à la première unité de communication (17) ;
l'appareil de surveillance (10) comprenant un premier module de lecture audio (161) connecté électriquement à la première unité de communication (17), le premier module de lecture audio (161) étant configuré pour restituer le retour audio afin d'apaiser le nourrisson.
